# EUROPEAN PATENT APPLICATION

(11) **EP 3 348 217 A1**
(43) Date of publication of application: **18.07.2018**
(21) Application number: 17204832.4
(22) Date of filing: 01.12.2017
(51) Int. Cl.: A61B 17/80

(54) **PLATE FOR SYNTHESIS OF A BONE FRACTURE AND KIT COMPRISING SUCH A PLATE**

(30) Priority: 12.01.2017 IT 201700002652
(71) Applicant: 2B1 S.r.l., 20124 Milano (IT)
(72) Inventor: ERRICO, Carmine, 20124 Milano (MI) (IT)
(74) Representative: Tiburzi, Andrea

(57) **Abstract**

This invention relates to a plate (1) for synthesis of a bone fracture, a first side (3) being defined in said plate (1), said plate (1) comprising, on said first side (3), at least two projecting portions (6) shaped for resting, in use, on a fractured bone (11), between said at least two projecting portions (6) at least one cavity (7) being formed for, in use, receiving at least one portion of a bone fracture reduction instrument (10), in such a way that said plate (1) is applicable, in use, on said fractured bone (11) while said instrument (10) is in the reduction position. This invention also relates to a kit comprising that plate (1) and at least one bone fracture reduction clamp (10) comprising at least one removable gripping portion (14), for gripping a fractured bone (11).

## Description

This invention relates to an improved plate for synthesis of a bone fracture and also to a kit incorporating that plate.

In medical terminology, "synthesis", or rather "osteosynthesis" refers to the stabilization of a bone fracture using devices that are surgically inserted at the level of the fractured bone.

When preparing to surgically treat the problem of a bone fracture by implantation of a plate using the open method, the first aspect to consider in order to achieve a good result from a medical viewpoint is that of obtaining satisfactory reduction.

Also in medical terminology, the term "reduction" means restoring the shape and/or function of the bone to what it was before the fracture.

Normally, bone reductions are performed by medical personnel by using K-wires and dedicated reduction clamps.

The basic implantation techniques commonly used for healing bone fractures involve the following steps.

First, fracture reduction in the operating room by means of external manipulation and, then, where possible, the limbs are stabilized by means of external supports and gripping instruments.

The surgeon then makes an incision near the fracture and opens the site (open method) in order to directly verify the reduction: during this step the surgeon checks, visually and not using x-rays, the correct coupling of the bone fragments in terms of position, proximity and morphology, in order to guarantee correct reconstruction of the fractured bone segment.

At this point, after having achieved a stable reduction of the fracture, using gripping instruments (clamps) for coupling the bone and the plate, it is possible to go ahead, in sequence, with positioning of the previously selected plate, removal of the clamps and, finally, fixing of the plate using screws.

In use, these last three operating steps are particularly critical.

In fact, once a satisfactory reduction has been achieved, as indicated above, it is necessary to position the synthesis plate in the same zone of the fractured bone in which the reduction clamps are applied, which very often get in the way of correct positioning of the plate.

In order to carry out this delicate operation, it is now common practice to loosen the grip of the clamps on the fractured bone, so as to allow sliding and then positioning of the plate between the clamping ends of the clamps and the bone.

However, in most cases this technique results in loss of the correct reduction previously achieved and, therefore, the need to restore that reduction.

Moreover, it extends the time required for surgeries, consequently increasing anaesthesiologic risks for the patient, as well as increasing the fracture exposure times, with an increase in infection risks for the surgical site and therefore also for the patient.

This type of practice, commonly used by medical personnel, also involves an increase in checks using x-rays, and therefore means increased exposure to radiation both for the patient, but above all, for surgical staff, as well as a reduction in the optimum times for use of operating rooms, with a consequent rise in related costs.

Therefore, in light of the above, the aim of this invention is to provide an improved plate for synthesis of a bone fracture, which is such that it can be implanted on the fractured bone without having to move the instruments used for fracture reduction.

Another aim of the invention is to provide an improved plate for synthesis of a bone fracture, that has mechanical resistance properties such that they allow it to effectively support the bone in the relative movements after implantation.

Another aim of the invention is to provide a kit incorporating that plate, which allows the minimizing of patient physical trauma during surgery for implantation of the self-same plate.

Therefore, this invention specifically relates to a plate for synthesis of a bone fracture, a first side being defined in said plate, said plate comprising, on said first side, at least two projecting portions shaped for resting, in use, on a fractured bone, between said at least two projecting portions at least one cavity being formed for, in use, receiving at least one portion of a bone fracture reduction instrument, in such a way that said plate is applicable, in use, on said fractured bone while said instrument is in the reduction position.

Furthermore, according to the invention, in said plate at least two holes may be formed, wherein each hole of said at least two holes passes through a respective projecting portion of said at least two projecting portions in such a way as to open on said first side.

Advantageously, according to the invention, in said plate, in a position opposite said first side, a second side may be defined in which at least one bulge is formed at said at least one cavity.

Preferably, according to the invention, said at least two projecting portions comprise a respective concave end portion suitable for being rested, in use, on a fractured bone.

Also according to the invention, said at least two projecting portions may comprise a respective end portion suitable for being rested, in use, on a fractured bone, said respective end portion comprising an inert material for avoiding, in use, osseointegration between said fractured bone and said plate.

Preferably, according to the invention, said inert material is polyether ether ketone (PEEK).

Also according to the invention, a third side and a fourth side may be defined in said plate, and said at least one cavity may pass through said third side and said fourth side.

Advantageously, according to the invention, said plate may extend along a substantially straight preferred line.

This invention also relates to a kit comprising said plate and at least one bone fracture reduction clamp comprising at least one removable gripping portion, for gripping a fractured bone.

Preferably, according to the invention, said at least one clamp comprises at least one screw for removably coupling said at least one gripping portion to a remaining portion of said at least one clamp.

This invention will now be described, by way of example and without limiting its scope, with reference to the accompanying drawings which illustrate a preferred embodiment of it, in which:
Figure 1 is a first view of a plate for synthesis of a fracture according to this invention;
Figure 2 is a second view of the plate shown in Figure 1;
Figure 3 is a third view of the plate of Figures 1 and 2;
Figure 4 shows a first step relating to implantation of the plate of Figures 1 to 3;
Figure 5 shows a second step relating to implantation of the plate of Figures 1 to 3; and
Figure 6 shows a third step relating to implantation of the plate of Figures 1 to 3.

In the various figures similar parts are labelled with the same reference numerals.

With reference to the accompanying figures, the numeral 1 denotes a plate for synthesis of a fracture according to this invention.

That plate 1 extends along a preferred line of extension according to a longitudinal axis P.

The above-mentioned plate 1 also comprises an upper side 2, a lower side 3 opposite to said upper side 2, a first lateral side 4 and a second lateral side 5 opposite to the first lateral side 4.

On the lower side 3 there is a plurality of projecting portions 6 separated by a plurality of grooves 7, which are positioned substantially equidistant from each other and directed perpendicularly to a plane passing through the longitudinal axis P and orthogonally incident on the plate 1.

Specifically, each groove of said plurality of grooves 7 is a through groove, that is to say, leads to both of the above-mentioned lateral sides 4, 5 of the plate 1.

The lower surface 6' of each projecting portion 6 is concave to better adapt to the outer surface of the fractured bone on which it will have to rest.

In contrast, on the upper side 2, the plate 1 comprises a set of humps 8, that is to say bulges, at or rather above the above-mentioned grooves 7, so as to render substantially uniform the distribution of material in the plate 1 according to the longitudinal axis P.

The above-mentioned humps 8, like the grooves 7 below, are substantially equidistant from each other.

This particular shape of the plate 1, with the humps 8 directly above the grooves 7, guarantees a strong cross-section that is substantially uniform along the entire longitudinal dimension of the plate 1, and therefore considerable fatigue strength for the plate 1 itself.

That technical device is particularly advantageous above all in cases of diaphyseal application of the plate 1, where the latter is inevitably subject to bending moments of significant intensity.

A plurality of through holes 9 is also made in the plate 1. They pass through the projecting portions 6, each leading to the upper side 2 and to the lower side 3 of the plate 1.

The function of the through holes 9 is to allow the plate 1 to be fixed to the fractured bone using fixing screws 9' that are passed through the through holes 9 and screwed onto the bone.

Those fixing screws 9' may comprise, for example, countersunk- or ball-head compression screws, and/or plastic fixing screws, also known as "angular stability" screws.

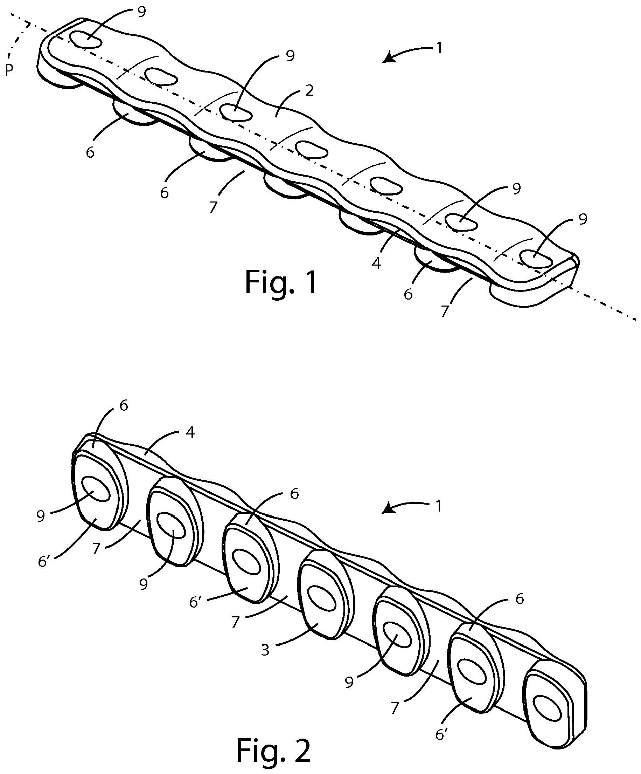

According to this invention, the plate described above may have a shape in plan view that is even different from the straight one shown in Figures 1 to 3, depending on the specific intended use of the plate, and more precisely based on the type of bone on which it must be applied.

The plates according to this invention may therefore comprise diaphyseal plates, anatomical plates for proximal, epiphyseal or distal approaches, fixed shape or mouldable plates, small or extremely small plates, if necessary implantable using sliding or minimally invasive techniques.

The plate 1 may be made, for example, of steel or its alloys, of titanium or its alloys, or of carbon, polyether ether ketone (PEEK) or other composite materials.

If the plate 1 is made of a material that is not inert, the lower surfaces 6' of the projecting portions 6 may be coated with an inert material for avoiding possible risks of osseointegration between the plate 1 and the fractured bone, which, should it occur, could make removal of the plate 1 from the bone once the fracture has fully healed particularly complicated.

An example of that coating material may be identified as polyether ether ketone (PEEK), which as is known is biologically inert and, therefore, is not subject to being colonized by bone tissue cells.

That plate 1 may be obtained, for example, by forging, mechanical stock removal, laser or water jet cutting, or by means of additive techniques, such as three-dimensional printing.

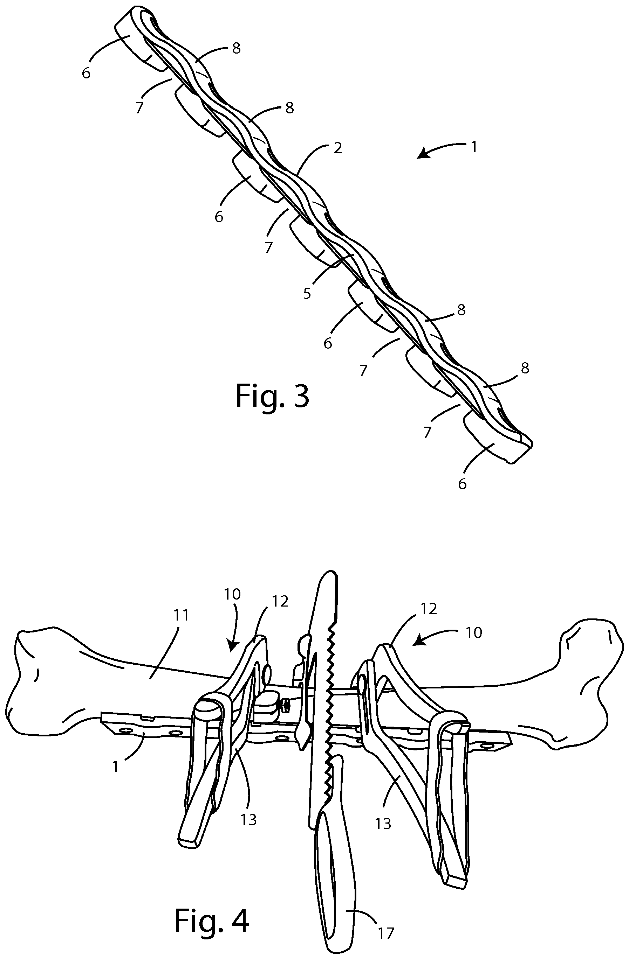

This invention also relates to a kit comprising, in addition to the plate described above, at least one clamp 10 for bone 11 fracture reduction (see Figure 4).

In particular, that clamp 10 comprises a first elongate element 12 and a second elongate element 13, which are hinged to each other at a respective intermediate point.

The first elongate element 12 comprises a removable end portion 14, also known as the "clamping end", intended to operate in conjunction with a corresponding end portion of the second elongate element 13 for gripping the bone 11.

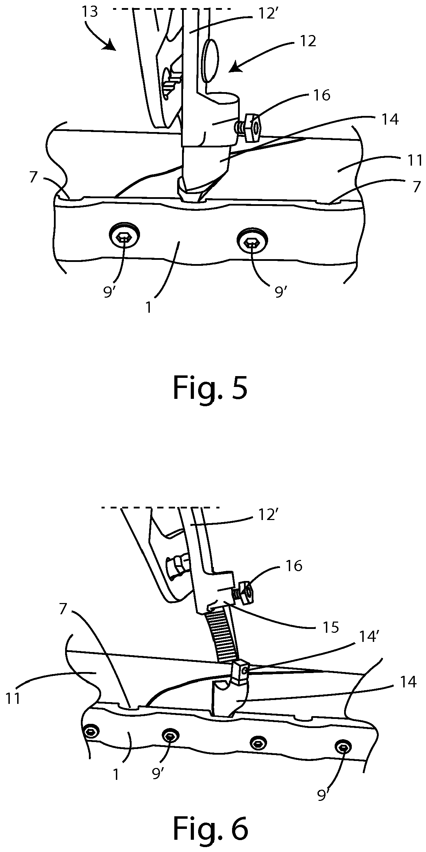

In the specific embodiment of the invention shown in Figures 4 to 6, the removable coupling between the removable end portion 14 and the remaining part 12' of the first elongate element 12 is obtained by having a cavity 15 at the end of said remaining part 12'.

In contrast, in an end zone of the removable end portion 14 there is a male connector 14' shaped to match the connector of said cavity 15, so that it can be housed inside the latter.

The removable end portion 14 is fixed in the cavity 15 thanks to a locking screw 16 that is screwed into a hole made in the above-mentioned remaining part 12' of the first elongate element 12 and communicating with the self-same cavity 15.

According to a variant of the clamp 10 described above, the second elongate element 13 also comprises a removable end portion similar to that of the first elongate element 12.

When, faced with a fractured bone, reduction of the fracture is required, it is possible to operate on the patient by gripping the bone with one or more clamps 10.

Once a stable reduction has been achieved, with the clamps 10 kept in the relative gripping position, the plate 1 is applied to the fractured bone 11 in such a way that the lower surfaces 6' of the projecting portions 6 rest directly on the bone 11 and that the removable end portion 14 of each clamp 10 is positioned inside a groove 7.

Therefore, the plate 1 is applied to be bone 11 while keeping the clamps 10 in the relative reduction position, in that way avoiding the risk of compromising the optimum reduction previously achieved.

After having rested the plate 1 on the fractured bone 11 in the way indicated above, the plate is held in position, for example with a normal clamp for medical use 17 that is positioned in such a way as to grip the plate 1 and the bone 11, for allowing fixing of the plate 1 to the bone 11 by screwing the screws 9' into the through holes 9.

Once fixing of the plate 1 on the bone 11 is complete, it is possible to remove the above-mentioned clamp for medical use 17 and the clamps 10 used for reduction of the fracture.

In order to minimize the physical trauma for the patient and avoid tissue damage, it is also possible, after having screwed the screws 9' on the bone 11, to separate the removable end portion 14 from the remaining part 12' of the first elongate element 12 by loosening the screw 16 and, subsequently, to remove the removable end portion 14 from the relative groove 7 made in the plate 1.

If, during the surgery, it should become necessary to apply temporary or permanent cerclage bands and/or wires and/or metal cerclage parts, the plate 1 could be positioned in such a way that even such bands and/or wires and/or metal parts are received in the grooves 7 of the plate 1 without interfering with the latter.

This invention is described by way of example only, without limiting the scope of application, according to its preferred embodiments, but it shall be understood that the invention may be modified and/or adapted by experts in the field without thereby departing from the scope of the inventive concept, as defined in the claims herein.

## Claims

1. A plate (1) for synthesis of a bone fracture, a first side (3) being defined in said plate (1), said plate (1) comprising, on said first side (3), at least two projecting portions (6) shaped for resting, in use, on a fractured bone (11), between said at least two projecting portions (6) at least one cavity (7) being formed for, in use, receiving at least one portion of a bone fracture reduction instrument (10), in such a way that said plate (1) is applicable, in use, on said fractured bone (11) while said instrument (10) is in the reduction position.

2. The plate (1) according to claim 1, **characterized in that** in said plate (1) at least two holes (9) are formed, wherein each hole of said at least two holes (9) passes through a respective projecting portion of said at least two projecting portions (6) in such a way as to open on said first side (3).

3. The plate (1) according to either of the preceding claims, **characterized in that** in said plate (1), in a position opposite said first side (3), a second side (2) is defined in which at least one bulge (8) is formed at said at least one cavity (7).

4. The plate (1) according to any of the preceding claims, **characterized in that** said at least two projecting portions (6) comprise a respective concave end portion (6') suitable for being rested, in use, on a fractured bone (11).

5. The plate (1) according to any of the preceding claims, **characterized in that** said at least two projecting portions (6) comprise a respective end portion (6') suitable for being rested, in use, on a fractured bone (11), said respective end portion (6') comprising an inert material for avoiding, in use, osseointegration between said fractured bone (11) and said plate (1).

6. The plate (1) according to claim 5, **characterized in that** said inert material is polyether ether ketone (PEEK).

7. The plate (1) according to any of the preceding claims, **characterized in that** a third side (4) and a fourth side (5) are defined in said plate (1), and **in that** said at least one cavity (7) passes through said third side (4) and said fourth side (5).

8. The plate (1) according to any of the preceding claims, **characterized in that** it extends along a substantially straight preferred line (P).

9. A kit comprising a plate (1) according to any of the preceding claims and at least one bone fracture reduction clamp (10) comprising at least one removable gripping portion (14), for gripping a fractured bone (11).

10. The kit according to the preceding claim, **characterized in that** said at least one clamp (10) comprises at least one screw (16) for removably coupling said at least one gripping portion (14) to a remaining portion (12') of said at least one clamp (10).
